# EUROPEAN PATENT APPLICATION

(11) **EP 0 755 686 A2**
(43) Date of publication of application: **29.01.1997**
(21) Application number: 96303007.7
(22) Date of filing: 30.04.1996
(51) Int. Cl.: A61L 2/06

(54) **On-line steam sanitization system**

(30) Priority: 01.05.1995 US 432927
(71) Applicant: JOHNSON & JOHNSON VISION PRODUCTS, INC., Jacksonville, Florida 32216-0995 (US)
(72) Inventor: Pricer, Kenneth Kurt, Jacksonville, Florida 32259 (US); Keene, Darren Scott, Jacksonville, Florida 32257 (US); Wang, Daniel Tsu-Fang, Jacksonville, Florida 32225 (US)
(74) Representative: Mercer, Christopher Paul

(57) **Abstract**

An on-line steam sanitization system and method for a deionized water supply system for a plant production line such as a plant for the production and packaging of contact lenses. The sanitizing operation is performed by directing a clean deionized steam through all of the deionized water supply system while the deionized water supply system is closed, rather than disassembled and open to possible recontamination as in the prior art, which provides a much more effective sanitization operation, and also substantially reduces the down time for the plant production line. The clean steam is generated by using available house steam to heat deionized water from the deionized water supply to generate a clean steam therefrom which is suitable for the sanitization operation. The use of steam in an on-line sanitizing operation in the existing deionized water supply system required that the production line equipment be modified to withstand the pressure and heat of the steam sanitizing operation, which entailed the removal of reservoirs and their replacement with pressure regulators, a new manifold design which has a symmetrical design with an equal number of fixtures leading to each of the pumps, and by utilizing Kynar tubes and Kynar fixtures.

## Description

### BACKGROUND OF THE INVENTION

### 1. Field of the Invention

The present invention relates generally to an on-line steam sanitization system and method. More particularly, the subject invention pertains to an on-line steam sanitization system and method for sanitizing a production plant deionized water supply system, such as in a plant for manufacturing and packaging contact lenses.

The present invention relates to sanitization processes, which are related to sterilization processes. Sterilization is a process by which objects, materials or environments may be rendered sterile, which refers to the condition of an object, or an environment, which is free of all living cells, all viable spores (and other resistant and disseminative forms), and all viruses and subviral agents capable of replication. An effective sterilization process must therefore irreversibly inactivate all organisms. For most practical purposes the main criterion of sterility is satisfied by the failure to detect viable cells, spores, viruses, etc. on or in a given object, material or environment. Methods of sterilization include physical methods such as heat, including dry heat and moist heat, radiation, pulsed energy such as light waves, filtration, ultrasonication, chemical methods, and also combined physical and chemical methods.

The present invention employs and relates to an on-line sanitization process and method, which in a contact lens manufacturing and packaging plant makes the equipment as clean as possible, eliminating microbes. However, in this environment the validation effort required to guarantee sterility is not economically viable, and accordingly the process is referred to as a sanitization process rather than a sterilization process. A target bioburden is below 1000 organisms per cubic liter.

### 2. Discussion of the Prior Art

Some plant production lines, such as those for the production and packaging of contact lenses, have a separate deionized water supply system with deionized water pumps, supply lines, and related equipment which supply deionized water to various areas of the plant which require deionized water for the operations being performed thereat.

Deionized water is generally distilled water which has all minerals and chemical additives removed therefrom, and is particularly suitable for immersing contact lenses therein during manufacturing to allow chemicals therein to leach out, and also for transferring lenses from one processing step to the next. The deionized water and an added surfactant allow the lenses to equilibrate rapidly, reach an equilibrium state in which the parameters of the lens do not change, as is desirable for lens transfer and lens inspection.

Unfortunately, the deionized water supply does not inhibit normal microbe buildup resultant from, among other factors, the open air assembly of the parts of the deionized water supply, and the added surfactant which supports the life of the microbes. Accordingly, the system has to be sanitized periodically, such as once every several weeks. Sanitization entailed, inter alia, disassembling the deionized water supply system positive displacement pumps, which typically dispense microliter dosages of deionized water, and replacing tubing, filters, etc. which typically might take four persons, each working 12-20 hours, for a total of 48-80 man hours. During this operation, the disassembled equipment which is returned to the deionized water supply system is sanitized in a sterilizer, which could be a steam and air mixture in an autoclave, or any suitable sterilizer. Equipment such as pumps, reservoirs and filters were sanitized in a sterilizer in which steam was injected into the equipment without disassembly of the equipment.

One disadvantage of the prior art approach to sanitizing the deionized water supply system was the possible contamination of the sanitized components by contact with the operator's hands and other unclean surfaces during assembly, and also necessary down time for the production line, which was down during the approximately 12-20 hour period, while the sanitizing operation was being performed, and this on a production line normally running 24 hours a day.

### SUMMARY OF THE INVENTION

Accordingly, it is a primary object of the present invention to provide an on-line steam sanitization system and method for a deionized water supply system.

A further object of the subject invention is the provision of a sanitizing operation which is performed through all of the deionized water supply system while the deionized water supply system is closed, rather than disassembled and open to possible recontamination as in the prior art sanitization process, which provides a much more effective sanitization operation than in the prior art, and also substantially reduces the down time for the plant production line.

It would be desirable to sanitize the deionized water supply system on a production line in an on-line manner by using steam directed therethrough, rather than having to disassemble the equipment, sanitize the disassembled equipment, and then reinstall it as in the prior art. The use of steam in an on-line sanitizing operation in the existing deionized water supply system required that the production line equipment be modified to withstand the pressure and heat of the steam sanitizing operation. One such modification entailed the removal of reservoirs, which could not withstand the pressure of sanitization, and their replacement with pressure regulators. The pressure regulators reduce the pressure of the sanitizing clean steam from approximately 40 psi to approximately 15 psi, which the positive displacement pumps can handle during the sanitization procedure. During normal operations, the pressure regulators reduce the pressure of the deionized water from approximately 10 psi to approximately 1-3 psi which the positive displacement pumps can handle.

The plant production line has a deionized water supply system available therein, and also has house steam available therein. House steam contains chemical additives which make it unsuitable for an on-line steam sanitization operation. Instead, pursuant to the present invention, the house steam is used to heat deionized water from the deionised water supply to generate a clean steam therefrom which is suitable for the sanitization operation on the deionized water supply system.

One major advantage of the present invention is that the sanitizing operation is performed through all of the deionized water supply system while the deionized water supply system is closed, rather than disassembled and open to possible recontamination as in the prior art sanitization process. The result is a much more effective sanitization. With the prior art open sanitization approach, the deionized water supply system sometimes repopulated with microbes within 72 hours, as evidenced by the bioburden thereof. With the closed sanitization approach of the present invention, sometimes the deionized water supply system evidences no repopulation with microbes between periodic maintenance sanitization operations.

A preventative maintenance sanitization operation pursuant to the present invention reduces the down time for the plant production line to approximately 2 hours, compared to 12-20 hours as in the prior art, and performs a much more effective sanitization operation than in the prior art, often with no microbe repopulation between periodic maintenance sanitization operations, as evidenced by the bioburden thereof.

The present invention introduces pressure regulation into the deionized water supply system, and also utilizes a new manifold design. The old prior art manifold used 16 tubes, one to each pump. The new manifold has a symmetrical design with an equal number of fixtures leading to each of the 16 pumps. The pumps have also been changed from Kynar pump bodies to stainless steel pump bodies to withstand the heat and pressure associated with steam.

In accordance with the teachings herein, the present invention provides an on-line steam sanitizing system and method for a production plant deionized water supply system, which includes deionized water pumps, supply lines, and related equipment for supplying deionized water to various areas of the production plant which require deionized water for the operations being performed thereat. The deionized water supply is sanitized by an on-line steam sanitization procedure in which steam is introduced into and through the deionized water supply, without disassembly thereof for a minimum sanitization period, after which the production plant returns to normal operation.

In greater detail, the on-line steam sanitizing system and method was developed for a plant for the production and packaging of contact lenses. The plant includes a predose facility wherein predoses of deionized water are supplied to packages making them ready to accept lenses to be deposited therein, and to a lens transfer facility wherein measured doses of deionized water are supplied to nozzles on robotic arms for transferring lenses from one processing step to the next.

The plant production line also has house steam available therein, which contains chemical additives which make it unsuitable for an on-line steam sanitization operation. Accordingly, the house steam is used to heat deionized water from the deionized water supply to generate a clean steam therefrom which is suitable for the sanitization operation.

The use of steam in a sanitization operation in an existing plant deionized water supply system required that the production line equipment be modified to withstand the pressure and heat of the steam sanitizing operation. The modifications included removing reservoirs, which provided an open environment and also could not withstand the pressure and heat of sanitization, and replacing the reservoirs with pressure regulators. The pressure regulators reduce the pressure of the sanitizing clean steam from approximately 40 psi to approximately 15 psi during the sanitization procedure, which the positive displacement pumps can handle. During normal operations, the pressure regulators reduce the pressure of the deionized water from approximately 10 psi to approximately 1-3 psi which the positive displacement pumps can handle. The modifications further include utilizing a new manifold design which has a symmetrical design with an equal number of fixtures leading to each of the plurality of pumps, and utilizing Kynar tubes and Kynar fixtures.

The deionized water is introduced into the deionized water supply system through a shut-off valve, which is closed during the steam sanitization operation, and opened thereafter. The clean steam is also introduced into the deionized water supply through a shut-off valve, which is opened during the steam sanitization operation, and is closed thereafter.

### BRIEF DESCRIPTION OF THE DRAWINGS

The foregoing objects and advantages of the present invention for an on-line steam sanitization system and method may be more readily understood by one skilled in the art with reference being had to the following detailed description of several preferred embodiments thereof, taken in conjunction with the accompanying drawings wherein like elements are designated by identical reference numerals throughout the several views, and in which:
Figure 1 illustrates a piping schematic diagram for the deionized water supply of a plant production line for the production and packaging of contact lenses.
Figure 2 is a more detailed piping schematic of the deionized water supply for a plant production line for the production and packaging of contact lenses;
Figure 3 is a detailed piping diagram for the deionized water supply of a predose facility; and
Figure 4 is a detailed piping diagram for the deionized water supply of a lens transfer facility.

### DETAILED DESCRIPTION OF THE DRAWINGS

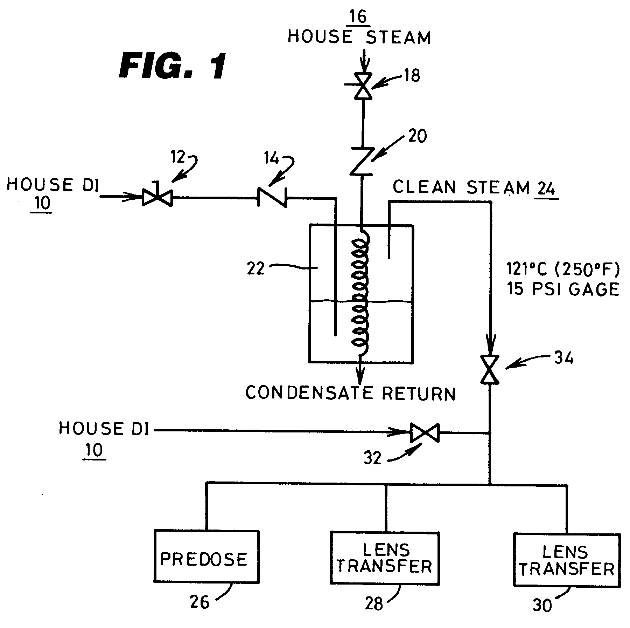

Referring to the drawings in detail, Figure 1 illustrates a piping schematic diagram for the deionized water supply for a plant production line for the production and packaging of contact lenses. The plant production line is normally supplied with deionized (DI) water at 10. Deionized water is generally distilled water which has all minerals and chemical additives removed therefrom, and is particularly suitable with an added surfactant for immersing contact lenses therein during manufacturing to allow chemicals therein to leach out and equilibrate quickly, and for transferring lenses from one processing step to the next. The deionized water allows the lenses to equilibrate, reach an equilibrium state in which the parameters of the lens do not change, as is desirable for lens transfer and lens inspection.

The plant production line is also normally supplied with house steam at 16 through a ball valve 18 and check valve 20. Unfortunately, house steam contains chemical additives which make it unsuitable for an on-line steam sanitization operation. Instead, pursuant to the present invention, the house steam is used in a DI steam generating vessel 22 to heat deionized water from the deionized water supply to generate a clean steam therefrom at 24 which is suitable for a sanitization operation on the deionized water supply system. The DI clean steam 24 is supplied at approximately 121°C at 15 psi gage.

The plant production line illustrated in Figure 1 is for the production and packaging of contact lenses, and includes three facilities 26, 28 and 30 which require deionized water in their operations. A predose facility 26 supplies predoses of deionized water to packages making them ready to accept lenses to be deposited therein. The lens transfer facilities 28 and 30 supply deionized water to nozzles on robotic arms for transferring lenses from one processing step to the next in operations performed between hydration and packaging in post hydration.

During normal operation of the plant production line, the predose facility 26 and the lens transfer facilities 28 and 30 are supplied with deionized water from the house deionized water supply 10 through a valve 32, which must be sanitized periodically, such as once every several weeks.

Accordingly, periodically such as every three weeks, the plant production line is shut down, and the production plant deionized water supply is sanitized by an on-line steam sanitization system as illustrated in Figure 1. Then, the house deionized water supply is shut off by valve 32, and clean steam is introduced, by opening valve 34, into and through the deionized water supply systems of the predose and lens transfer facilities 26, 28 and 30. The clean steam should flow through and sanitize the equipment for a minimum predetermined sanitization period, such as 30 minutes, and the entire operation of converting from production to sanitization and back to production takes one man approximately 2 hours, compared to 12-20 hours as in the prior art. Thus, the present invention results in a substantial decrease in lost production time of the plant.

Another advantage of the present invention is that the sanitizing operation is much cleaner and more effective as it is performed through all of the deionized water supply system while it is closed, rather than disassembled and open to possible recontamination as in the prior art sanitization process. The result is a much more effective sanitization process. With the prior art open sanitization approach, the deionized water supply system is sometimes repopulated with microbes within 72 hours, as evidenced by the bioburden thereof. With the closed sanitization approach of the present invention, sometimes the deionized water supply system evidences no repopulation with microbes between periodic maintenance sanitization operations.

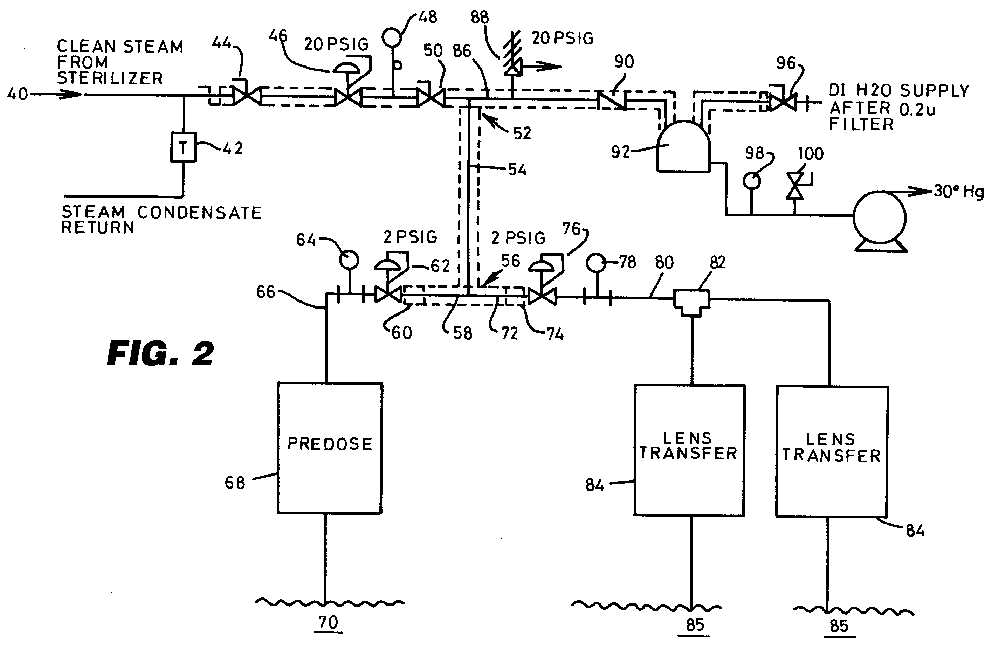

Figure 2 is a more detailed piping schematic of the deionised water supply for a plant production line for the production and packaging of contact lenses. During a sanitization operation, clean steam from a sterilizer as shown in Figure 1 is introduced at the upper left at 40, with condensate collecting in a steam trap 42. The clean steam flows through a ball valve 44, through steam pressure regulator 46, by pressure gage 48, through ball valve 50, through a T fitting 52, in one leg 54 downwardly to a further T fitting 56, in one leg 58 to the left, through an adaptor 60, 3/8" comp. to 1/2" sanitary, pressure regulator 62, by a pressure gage 64, through an adaptor 66, 3/8" comp to 1/2" sanitary, to a predose facility 68 which feeds 16 predose nozzles or needles 70. Returning to T 56, the right leg 72 leads to an adaptor 74, 3/8" comp to 1/2" sanitary, pressure regulator 76, gage 78, an adaptor 80, 3/8" comp to 1/2" sanitary, to a Kynar T 82, and then to both lens transfer facilities 84, each of which feeds 16 transfer nozzles 85. Returning to T 52, the right leg 86 flows to a pressure relief valve 88, a check valve 90, and a degasser 92, which is a vacuum vessel to degas the deionized water during normal production, up to a ball valve 96. The vacuum degasser is supplied with a vacuum by a vacuum pump 98, operating through a vacuum relief valve 100, and a pressure gage 102.

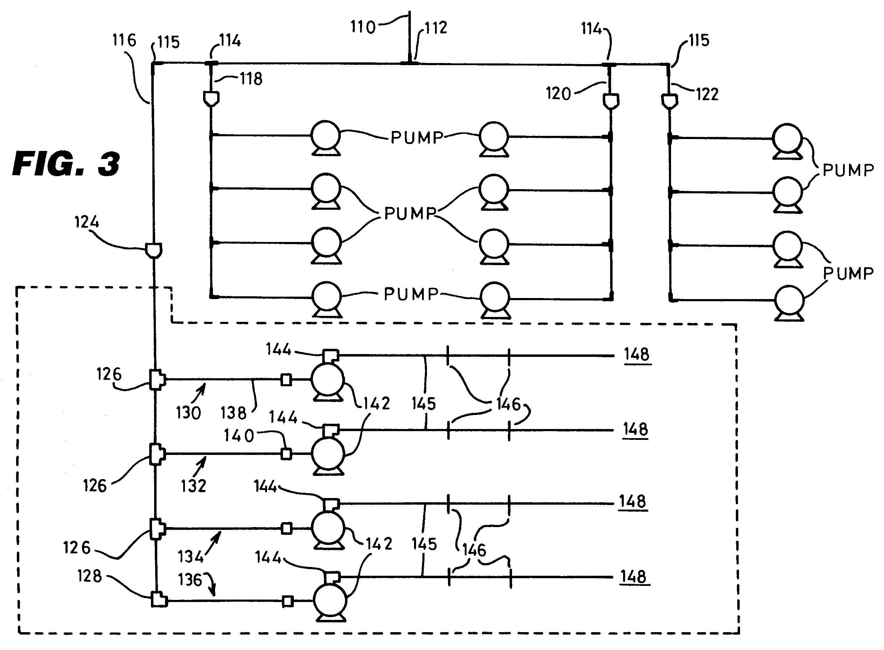

Figure 3 is a piping detail diagram for the deionised water supply of a predose facility. In Figure 3, all fittings and tubing are Kynar, and all fittings have steel grippers. Fluid (either deionized water during normal operation or clean steam during a sanitizing operation) flows through a central tube 110, Kynar tubing 3/8" OD x 1/4" ID, to a 3/8" Kynar T 112, and then divides to the left to one additional 3/8" Kynar T 114, and one additional 3/8" Kynar L 115, and also divides to the right to one additional 3/8" Kynar T 114, and one additional 3/8" Kynar L 115.

The arrangement includes four symmetrical legs 116, 118, 120 and 122, one leg depending from each T 114 or L 115. Only the left-most leg 116 of the four symmetrical legs 116 is illustrated in detail in Figure 3, and it will be explained in detail with the understanding that the other three legs 118, 120 and 122 are substantially identical. Fluid flows through the 3/8" L 115 to a 3/8"-1/4" reducer 124 and then to three successive 1/4" Ts 126 and a last 1/4" L 128. Fluid then flows to the right (in Figure 3) in four substantially identical legs 130, 132, 134 and 136. Each leg includes a 1/4" tube 138, a union 140, a positive displacement pump 142, the output of which flows through a 1/4" L 144, through a 1/4" OD x 1/8" ID Kynar tube 145, through locks 146 (manufactured by Luer), which are essentially medical fittings, to Teflon or stainless steel nozzles or needles 148, through which a dosage of deionized water is injected into a package or container for handling a contact lens therein.

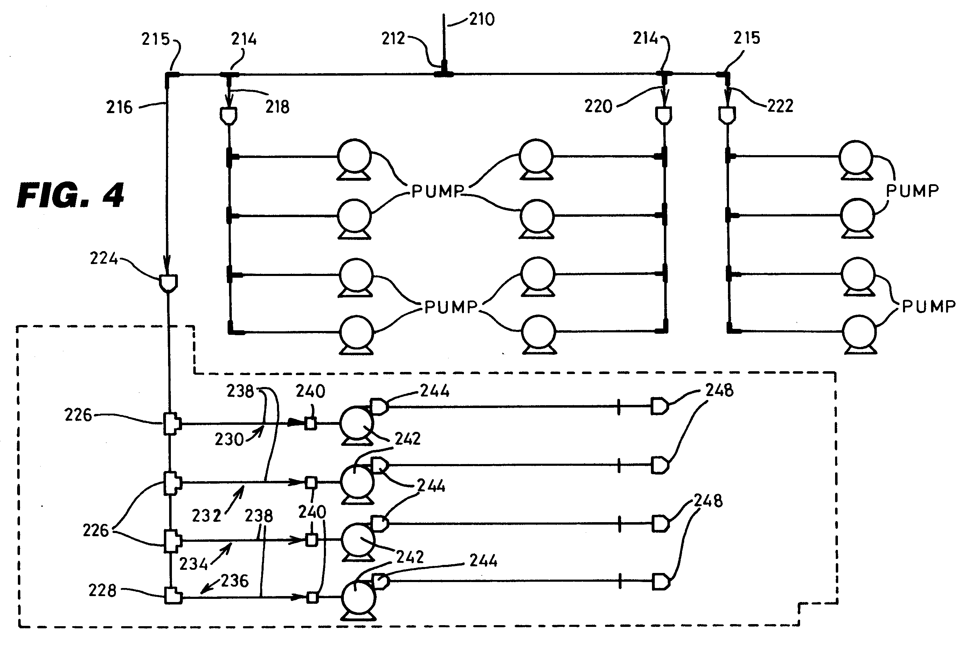

Figure 4 is a piping detail diagram of the deionized water supply of a lens transfer facility. In Figure 3, all fittings and tubing are Kynar, and all fittings have steel grippers. Fluid (either deionized water during normal operation or clean steam during a sanitizing operation) flows into a central tube 210, Kynar tubing 3/8" OD x 1/4" ID, to a 3/8" Kynar T 212, and then divides to the left to one additional 3/8" Kynar T 214, and one additional 3/8" Kynar L 215, and also divides to the right to one additional 3/8" Kynar T 214, and one additional 3/8" Kynar L 215. The arrangement includes four symmetrical legs 216, 218, 220 and 222, one leg depending from each L 215 or T 214. Only the left-most 216 of the four symmetrical legs is illustrated in detail in Figure 3, and it will be explained in detail with the understanding that the other three legs 218, 220 and 222 are substantially identical. Fluid flows through the 3/8" L 215 to a 3/8"-1/4" reducer 224 and then to three successive 1/4" Ts 226 and a last 1/4" L 228. Fluid then flows to the right (in Figure 4) in four substantially identical legs 230, 232, 234 and 236. Each leg includes a 1/4" tube 238, a union 240, a positive displacement pump 242, the output of which flows through a 1/4" x 1/8" reducer 244, through a 1/8" OD x 1/16" ID tube 246, to lens transfer nozzles 248, through which a dosage of deionized water is injected into a package or container for transferring lenses from one processing step to the next.

While several embodiments and variations of the present invention for an on-line steam sanitization system and method are described in detail herein, it should be apparent that the disclosure and teachings of the present invention will suggest many alternative designs to those skilled in the art.

## Claims

1. An on-line steam sanitizing method which comprises, supplying deionized water through a production plant deionized water supply system, which includes deionized water pumps, supply lines, and related equipment, to supply deionized water to various areas of the production plant which require deionized water for the operations being performed thereat, and sanitizing the production plant deionized water supply by an on-line steam sanitization procedure in which steam is introduced into and through the deionized water supply, without disassembling the deionized water supply system, for a predetermined sanitization period, after which the production plant is returned to normal operation.

2. An on-line steam sanitizing method for a production plant as claimed in claim 1, wherein the deionized water is supplied to a production plant which produces and packages contact lenses.

3. An on-line steam sanitizing method for a production plant as claimed in claim 2, wherein the deionized water is supplied to a predose facility for supplying predoses of deionized water to packages making them ready to accept lenses to be deposited therein, and to a lens transfer facility for supplying measured doses of deionized water to nozzles on robotic arms for transferring lenses from one processing step to the next.

4. An on-line steam sanitizing method for a production plant as claimed in claim 2, wherein a source of house steam heats deionized water from the deionized water supply to generate a clean deionized steam therefrom, and the clean deionized steam is used in the sanitizing step.

5. An on-line steam sanitizing method for a production plant as claimed in claim 1, wherein the use of steam in a sanitizing operation in an existing deionized water supply system required modifying the production plant equipment to withstand the pressure and heat of the steam sanitizing step.

6. An on-line steam sanitizing method for a production plant as claimed in claim 5, wherein said modifying step includes removing reservoirs, which could not withstand the pressure and heat of sanitization, and replacing the reservoirs with pressure regulators.

7. An on-line steam sanitizing method for a production plant as claimed in claim 6, including reducing the pressure of the sanitizing clean steam from approximately 10 psi to approximately 1-3 psi with the pressure regulators.

8. An on-line steam sanitizing method for a production plant as claimed in claim 5, wherein said modifying step includes utilizing a new manifold design which has a symmetrical design leading to each of the pumps.

9. An on-line steam sanitizing method for a production plant as claimed in claim 8, including utilizing in the symmetrical design an equal number of fixtures leading in each line to each of a plurality of pumps in a symmetrical arrangement.

10. An on-line steam sanitizing method for a production plant as claimed in claim 5, wherein the modifying step includes utilizing Kynar tubes and Kynar fixtures.

11. An on-line steam sanitizing method for a production plant as claimed in claim 3, including introducing deionized water into the deionized water supply system through a shut-off valve, and closing the deionized water shut-off valve during the steam sanitizing step, after which it is opened, and introducing the clean steam into the deionized water supply through a shut-off valve, and opening the clean steam shut-off valve during the steam sanitization operation, after which it is closed.

12. An on-line steam sanitizing method for a production plant as claimed in claim 1, wherein a source of house steam heats deionized water from the deionized water supply to generate a clean deionized steam therefrom, and the clean deionized steam is used in the sanitizing step.

13. An on-line steam sanitizing method for a production plant as claimed in claim 1, including introducing deionized water into the deionized water supply system through a shut-off valve, and closing the deionized water shut-off valve during the steam sanitizing step, after which it is opened, and introducing the clean steam into the deionized water supply through a shut-off valve, and opening the clean steam shut-off valve during the steam sanitization operation, after which it is closed.

14. An on-line steam sanitizing system for a production plant deionized water supply system which includes deionized water pumps, supply lines, and related equipment, to supply deionized water to various areas of the production plant which require deionized water for the operations being performed thereat, and a sanitizing steam input to the production plant deionized water supply, which is sanitized by an on-line steam sanitization procedure in which sanitizing steam is introduced from the sanitizing steam input into and through the deionized water supply, without disassembly thereof, for a predetermined sanitization period, after which the production plant is returned to normal operation.

15. An on-line steam sanitizing system for a production plant as claimed in claim 14, wherein the production plant includes facilities for the production and packaging of contact lenses.

16. An on-line steam sanitizing system for a production plant as claimed in claim 15, wherein the plant production line facilities include a predose facility wherein predoses of deionized water are supplied to packages making them ready to accept lenses to be deposited therein, and a lens transfer facility, which includes nozzles on robotic arms, to which measured doses of deionized water are supplied for transferring lenses from one processing step to the next.

17. An on-line steam sanitizing system for a production plant as claimed in claim 15, wherein the production plant facilities include a source of house steam, and a sanitizing vessel in which house steam is used to heat deionized water from the deionized water supply to generate a clean deionized steam therefrom which is suitable for the sanitization procedure.

18. An on-line steam sanitizing system for a production plant as claimed in claim 14, wherein the use of steam in a sanitizing operation in an existing deionized water supply system required that the production plant equipment be modified to withstand the pressure and heat of the steam sanitizing operation, and the modifications include the addition of pressure regulators which reduce the pressure of the sanitizing clean steam or distilled water.

19. An on-line steam sanitizing system for a production plant as claimed in claim 18, wherein the modifications include a manifold which has a symmetrical design leading to each of a plurality of pumps.

20. An on-line steam sanitizing system for a production plant as claimed in claim 19, wherein the symmetrical design includes an equal number of fixtures leading in a symmetrical arrangement to each of the plurality of pumps.

21. An on-line steam sanitizing system for a production plant as claimed in claim 18, wherein the modifications include Kynar tubes and Kynar fixtures.

22. An on-line steam sanitizing system for a production plant as claimed in claim 16, including a shut-off valve, through which deionized water is introduced into the deionized water supply system, which is closed during the steam sanitization operation and is opened thereafter, and a second shut-off valve, through which clean steam is introduced into the deionized water supply, which is opened during the steam sanitization operation and is closed thereafter.

23. An on-line steam sanitizing system for a production plant as claimed in claim 14, wherein the production plant facilities include a source of house steam, and a sanitizing vessel in which house steam is used to heat deionized water from the deionized water supply to generate a clean deionized steam therefrom which is suitable for the sanitization procedure.

24. An on-line steam sanitizing system for a production plant as claimed in claim 14, including a shut-off valve, through which deionized water is introduced into the deionized water supply system, which is closed during the steam sanitization operation and is opened thereafter, and a second shut-off valve, through which clean steam is introduced into the deionized water supply, which is opened during the steam sanitization operation and is closed thereafter.
